(19) **Europäisches Patentamt**
**European Patent Office**
**Office européen des brevets**

(11) **EP 3 489 342 B1**

(12) **EUROPEAN PATENT SPECIFICATION**

(45) Date of publication and mention
of the grant of the patent:
**26.01.2022 Bulletin 2022/04**

(51) International Patent Classification (IPC):
*C12M 1/00* (2006.01)          *C12N 1/00* (2006.01)
*C12M 1/12* (2006.01)

(21) Application number: **17834361.2**

(52) Cooperative Patent Classification (CPC):
**C12M 29/12; C12M 1/00; C12M 25/02; C12N 1/00**

(22) Date of filing: **25.07.2017**

(86) International application number:
**PCT/JP2017/026939**

(87) International publication number:
**WO 2018/021359 (01.02.2018 Gazette 2018/05)**

(54) **SIPHON TYPE CULTIVATION METHOD**

SIPHONARTIGES KULTIVIERUNGSVERFAHREN

PROCÉDÉ DE CULTURE DE TYPE SIPHON

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.07.2016 JP 2016145809**

(43) Date of publication of application:
**29.05.2019 Bulletin 2019/22**

(73) Proprietor: **UBE Industries, Ltd.
Ube-shi, Yamaguchi 755-8633 (JP)**

(72) Inventors:
• **HAGIHARA, Masahiko
Ube-shi
Yamaguchi 755-8633 (JP)**

• **FUSE, Shinsaku
Ube-shi
Yamaguchi 755-8633 (JP)**

(74) Representative: **Plougmann Vingtoft a/s
Strandvejen 70
2900 Hellerup (DK)**

(56) References cited:
**EP-A1- 3 026 108          EP-A1- 3 489 348
EP-A1- 3 489 351          WO-A1-2015/012415
JP-A- H03 195 486          JP-A- 2006 035 024
JP-A- 2014 100 097**

**Description**

FIELD

The present invention relates to a cell culture device comprising a porous polymer film, wherein the medium is intermittently discharged into the medium recovery means and further relates to a cell culture method using the cell culture device comprising the porous polymer film.

BACKGROUND

In recent years, proteins such as enzymes, hormones, antibodies, cytokines, viruses (viral proteins) used for treatment and vaccine are industrially produced using cultured cells. However, such a protein production technology is expensive, raising medical cost. Accordingly, there have been demands for innovating technologies for culturing cells at high density and for increasing protein production, aiming at great reduction of cost.

As cells for protein production, anchorage-dependent adherent cells which adhere to a culture substrate may be sometimes used. Since such cells grow anchorage-dependently, they need to be cultured while being adhered onto the surface of a dish, plate or chamber. Conventionally, in order to culture such adherent cells in a large amount, it was preferable to increase the surface area to be adhered. However, increasing the culturing area inevitably requires to increase the space, which is responsible for increase in cost.

As a method to culture a large amount of adherent cells while decreasing the culture space, a method for culture using a microporous carrier, especially a microcarrier, has been developed (for example, PTL 1). In a cell culturing system using microcarriers, it is preferable to carry out sufficient stirring and diffusion so that the microcarriers do not aggregate together. Since this requires a volume allowing adequate agitation and diffusion of the medium in which the microcarriers are dispersed, there is an upper limit to the density at which the cells can be cultured. In order to separate the microcarrier from the medium, separation is preferably performed using a filter which can separate fine particles, possibly resulting in increased cost. Considering the foregoing, there is a demand for innovative methodology for cell culture which cultures cells at high density.

<Porous Polyimide Film>

Porous polyimide films have been utilized in the prior art for filters and low permittivity films, and especially for battery-related purposes, such as fuel cell electrolyte membrane and the like. PTLs 2 to 4 describe porous polyimide films with numerous macrovoids, having excellent permeability to objects such as gases, high porosity, excellent smoothness on both surfaces, relatively high strength and, despite high porosity, excellent resistance against compression stress in the film thickness direction. All of these are porous polyimide films formed via amic acid.

The cell culture method which includes applying cells to a porous polyimide film and culturing them is reported (PTL 5).

[CITATION LIST]

[PATENT LITERATURE]

[PTL 1] WO2003/054174
[PTL 2] WO2010/038873
[PTL 3] Japanese Unexamined Patent Publication (Kokai) No. 2011-219585
[PTL 4] Japanese Unexamined Patent Publication (Kokai) No. 2011-219586
[PTL 5] WO2015/012415

SUMMARY

[TECHNICAL PROBLEM]

The object of the present invention is to provide a cell culture device comprising a porous polymer film and to provide a cell culture method using the cell culture device comprising the porous polymer film.

[SOLUTION TO PROBLEM]

The inventors of the present invention discovered that a porous polymer film having a predetermined structure not only provides an optimal space in which a large number of cells can be cultured, but also provides a moist environment that is resistant to drying and completed an device that cultures cells by the exposure thereof to a gas phase and a culture method using same. In other words, the present invention refers to the following modes.

[1] A cell culture device characterized by comprising:

a porous polymer film;
a cell culture part that accommodates the porous polymer film;
a liquid storage part that stores the cell culture part therein;
a medium supply means disposed on an upper part of the liquid storage part;
an inverted U-shaped tube communicating with a bottom part of the liquid storage part;
a medium recovery means provided below the other end of the inverted U-shaped tube; and
a medium discharge means disposed on the medium recovery means;
wherein the porous polymer films are a three-layer structure porous polymer film having a surface layer A and a surface layer B, the surface layers having a plurality of pores, and a macrovoid layer sandwiched between the surface layers A and B;
wherein an average pore diameter of the pores present in the surface layer A is smaller than an average pore diameter of the pores present in the surface layer B;
wherein the macrovoid layer has a partition wall bonded to the surface layers A and B, and a plurality of macrovoids surrounded by such a partition wall and the surface layers A and B;
wherein the medium supply means is set at a position higher than the cell culture part, and a top part of the inverted U-shaped tube is set at the same height as the top of the cell culture part or at a position lower than the medium supply means; and
wherein when the liquid surface of the medium supplied to the liquid storage part from the medium supply means reaches a top part of the inverted U-shaped tube, the medium is intermittently discharged into the medium recovery means due to the siphon principle.

[2] A further embodiment of the present invention is a cell culture device characterized by comprising:

a porous polymer film;
a box-shaped body, one end of which is a liquid storage part, the other end of which is a bottom part, provided with a cell culture part in the liquid storage part that accommodates the porous polymer film;
a rotary shaft provided in a part at the bottom surface side of the box-shaped body;
a bearing supporting the rotary shaft;
a medium supply means disposed above the liquid storage part; and
a medium recovery means disposed below the liquid storage part;
wherein the porous polymer films are a three-layer structure porous polymer film having a surface layer A and a surface layer B, the surface layers having a plurality of pores, and a macrovoid layer sandwiched between the surface layers A and B;
wherein an average pore diameter of the pores present in the surface layer A is smaller than an average pore diameter of the pores present in the surface layer B;
wherein the macrovoid layer has a partition wall bonded to the surface layers A and B, and a plurality of macrovoids surrounded by such a partition wall and the surface layers A and B; and
wherein when the medium supplied to the liquid storage part from the medium supply means exceeds the weight of the bottom part of the box-shaped body, the medium from the liquid storage part is intermittently discharged into the medium recovery means, and once the medium is discharged, the box-shaped body returns to the original horizontal position.

A method for culturing a cell which uses the aforementioned cell culture devices is also subject of the present invention.

[ADVANTAGEOUS EFFECTS OF INVENTION]

The present invention allows simple and efficient continuous cell culturing even under conditions requiring little space and little culture medium by using a porous polymer film as a cell culture support. Further, as the porous polymer film has pores with slightly hydrophilic properties, stable liquid retention is achieved within the porous polymer film and a moist environment that is resistant to drying is maintained. Thus, survival and proliferation of cells can be achieved even with very little medium compared to conventional cell culture devices. Furthermore, as culturing is possible even if a part of or the entire porous polymer film is exposed to air, oxygen can be efficiently supplied to the cells and a large number of cells can be cultured.

According to the present invention, a stable oxygen supply can be steadily achieved by directly exposing a cell culture part to a gaseous phase by a siphon mechanism or a "shishi odoshi" mechanism and thus a large number of cells can be cultured without the use of an oxygen supply system. Further, as the system spontaneously repeats movement of the liquid part, the operation thereof can be continued without the use of special equipment or energy other than for the flow of liquid. Furthermore, as the cells and a medium storage unit at a lower part (also referred to as "cell recovery means") are separate, when, for example, adjustments such as adjustments in pH or in the addition of glucose are made, even vigorous stirring, foaming and the like, which are disapproved of with respect to cells, can be performed. This allows for a greater number of options at the work facility, a release from theoretical upper limits to the scale of cell culture, and the possibility of developing extra-large facilities, etc.

BRIEF DESCRIPTION OF DRAWINGS

FIG. 1 is a diagram illustrating a siphon-type cell culture device according to an embodiment.

FIG. 2 is a diagram illustrating a "shishi odoshi"-type cell culture device according to an embodiment

FIG. 3(A) is a diagram illustrating a dry heat sterilization type siphon type cell culture device (heat-resistant siphon-type reactor) according to an embodiment and (B) is a diagram illustrating a module.

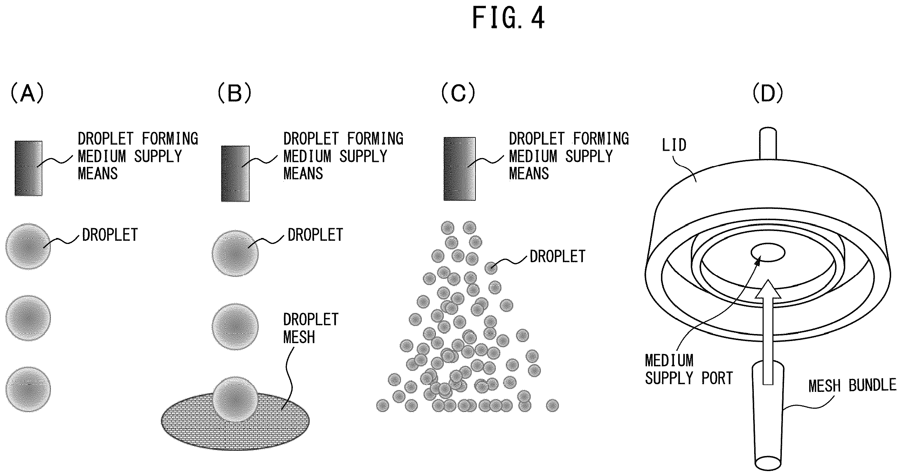

FIG. 4 is a conceptual diagram illustrating the form of a medium dripped from a droplet forming medium supply means in a cell culture device according to an embodiment. (A) illustrates a drop type, (B) illustrates a mesh type, and (C) illustrates a shower type. (D) is a diagram illustrating a lid used in a cell culture device according to one embodiment used for supplying droplets from the drop type and mesh type. A mesh bundle formed by winding up a stainless steel mesh is inserted into a medium supply port in the lid.

FIG. 5 is a graph illustrating the amount of fibronectin produced from human dermal fibroblasts when the cell culture device of the present invention is used in one embodiment. As a control, the amount of fibronectin produced from cultured human dermal fibroblasts in a normal culture dish is illustrated (dish day 8).

FIG. 6 shows a drawing of a cell culture model using a porous polymer film.

DETAILED DESCRIPTION OF INVENTION

The embodiments of the present invention will be described below referring to the drawings as needed.

1. Porous polymer film

An average pore diameter of the pore present on a surface layer A (hereinafter referred to as "surface A" or "mesh surface") in the porous polymer film used for the present invention is smaller than an average pore diameter of the pores present in the surface layer B; wherein an average pore diameter of the pores present in the surface layer A is, for example, 0.01 $\mu$m or more and less than 200 $\mu$m, 0.01 to 150 $\mu$m, 0.01 to 100 $\mu$m, 0.01 to 50 $\mu$m, 0.01 to 40 $\mu$m, 0.01 to 30 $\mu$m, 0.01 to 20 $\mu$m, or 0.01 to 15 $\mu$m, preferably 0.01 to 15 $\mu$m.

The average pore diameter of the pore present on a surface layer B (hereinafter referred to as "surface B" or "large pore surface") in the porous polymer film used for the present invention is not particularly limited so long as it is larger than the average pore diameter of the pore present on the surface A, but is, for example, greater than 5 $\mu$m and 200 $\mu$m or less, 20 $\mu$m to 100 $\mu$m, 30 $\mu$m to 100 $\mu$m, 40 $\mu$m to 100 $\mu$m, 50 $\mu$m to 100 $\mu$m, or 60 $\mu$m to 100 $\mu$m, preferably 20 $\mu$m to 100 $\mu$m.

The average pore diameter on the surface of the porous polymer film is determined by measuring pore area for 200 or more open pore portions, and calculated an average diameter according to the following Equation (1) from the average pore area assuming the pore shape as a perfect circle.

[Math. 1]

$$\text{Average Pore Diameter} = 2 \times \sqrt{(S \, a \, / \, \pi)} \qquad (1)$$

(wherein Sa represents the average value for the pore areas)

The thicknesses of the surface layers A and B are not particularly limited, but is, for example, 0.01 to 50 μm, preferably 0.01 to 20 μm.

The average pore diameter of macrovoids in the planar direction of the film in the macrovoid layer in the porous polymer film is not particularly limited but is, for example, 10 to 500 μm, preferably 10 to 100 μm, and more preferably 10 to 80 μm. The thicknesses of the partition wall in the macrovoid layer are not particularly limited, but is, for example, 0.01 to 50 μm, preferably 0.01 to 20 μm. In an embodiment, at least one partition wall in the macrovoid layer has one or two or more pores connecting the neighboring macrovoids and having the average pore diameter of 0.01 to 100 μm, preferably 0.01 to 50 μm. In another embodiment, the partition wall in the macrovoid layer has no pore.

The total film thickness of the porous polymer film used for the invention is not particularly limited, but may be 5 μm or more, 10 μm or more, 20 μm or more or 25 μm or more, and 500 μm or less, 300 μm or less, 100 μm or less, 75 μm or less, or 50 μm or less. It is preferably 5 to 500 μm, and more preferably 25 to 75 μm.

The film thickness of the porous polymer film used for the invention can be measured using a contact thickness gauge.

The porosity of the porous polymer film used in the present invention is not particularly limited but is, for example, 40% or more and less than 95%.

The porosity of the porous polymer film used for the invention can be determined by measuring the film thickness and mass of the porous film cut out to a prescribed size, and performing calculation from the basis weight according to the following Equation (2).

[Math. 2]

$$\text{Porosity}\,(\%) = (1 - w / (S \times d \times D)) \times 100 \qquad (2)$$

(wherein S represents the area of the porous film, d represents the total film thickness, w represents the measured mass, and D represents the polymer density. The density is defined as 1.34 g/cm³ when the polymer is a polyimide.)

The porous polymer film used for the present invention is preferably a porous polymer film which includes a three-layer structure porous polymer film having a surface layer A and a surface layer B, the surface layers having a plurality of pores, and a macrovoid layer sandwiched between the surface layers A and B; wherein the average pore diameter of the pore present on the surface layer A is 0.01 μm to 15 μm, and the average pore diameter of the pore present on the surface layer B is 20 μm to 100 μm; wherein the macrovoid layer has a partition wall bonded to the surface layers A and B, and a plurality of macrovoids surrounded by such a partition wall and the surface layers A and B, the thickness of the macrovoid layer, and the surface layers A and B is 0.01 to 20 μm; wherein the pores on the surface layers A and B communicate with the macrovoid, the total film thickness is 5 to 500 μm, and the porosity is 40% or more and less than 95%. In an embodiment, at least one partition wall in the macrovoide layer has one or two or more pores connecting the neighboring macrovoids with each other and having the average pore diameter of 0.01 to 100 μm, preferably 0.01 to 50 μm. In another not claimed incident the partition wall does not have such pores.

The porous polymer film used for the present invention is preferably sterilized. The sterilization treatment is not particularly limited, but any sterilization treatment such as dry heat sterilization, steam sterilization, sterilization with a disinfectant such as ethanol, electromagnetic wave sterilization such as ultraviolet rays or gamma rays, and the like can be mentioned.

The porous polymer film used for the present invention is not particularly limited so long as it has the structural features described above and includes, preferably a porous polyimide film or porous polyethersulfone film.

1-1. Porous polyimide film

Polyimide is a general term for polymers containing imide bonds in the repeating unit, and usually it refers to an aromatic polyimide in which aromatic compounds are directly linked by imide bonds. An aromatic polyimide has an aromatic-aromatic conjugated structure via an imide bond, and therefore has a strong rigid molecular structure, and since the imide bonds provide powerful intermolecular force, it has very high levels of thermal, mechanical and chemical properties.

The porous polyimide film usable for the present invention is a porous polyimide film preferably containing polyimide (as a main component) obtained from tetracarboxylic dianhydride and diamine, more preferably a porous polyimide film composed of tetracarboxylic dianhydride and diamine. The phrase "including as the main component" means that it essentially contains no components other than the polyimide obtained from a tetracarboxylic dianhydride and a diamine, as constituent components of the porous polyimide film, or that it may contain them but they are additional components that do not affect the properties of the polyimide obtained from the tetracarboxylic dianhydride and diamine.

In an embodiment, the porous polyimide film usable for the present invention includes a colored porous polyimide film obtained by forming a polyamic acid solution composition including a polyamic acid solution obtained from a tetracarboxylic acid component and a diamine component, and a coloring precursor, and then heat treating it at 250°C or higher.

A polyamic acid is obtained by polymerization of a tetracarboxylic acid component and a diamine component. A polyamic acid is a polyimide precursor that can be cyclized to a polyimide by thermal imidization or chemical imidization.

The polyamic acid used may be any one that does not have an effect on the invention, even if a portion of the amic acid is imidized. Specifically, the polyamic acid may be partially thermally imidized or chemically imidized.

When the polyamic acid is to be thermally imidized, there may be added to the polyamic acid solution, if necessary, an imidization catalyst, an organic phosphorus-containing compound, or fine particles such as inorganic fine particles or organic fine particles. In addition, when the polyamic acid is to be chemically imidized, there may be added to the polyamic acid solution, if necessary, a chemical imidization agent, a dehydrating agent, or fine particles such as inorganic fine particles or organic fine particles. Even if such components are added to the polyamic acid solution, they are preferably added under conditions that do not cause precipitation of the coloring precursor.

In this specification, a "coloring precursor" is a precursor that generates a colored substance by partial or total carbonization under heat treatment at 250°C or higher.

Coloring precursors usable for the production of the porous polyimide film are preferably uniformly dissolved or dispersed in a polyamic acid solution or polyimide solution and subjected to thermal decomposition by heat treatment at 250°C or higher, preferably 260°C or higher, even more preferably 280°C or higher and more preferably 300°C or higher, and preferably heat treatment in the presence of oxygen such as air, at 250°C, preferably 260°C or higher, even more preferably 280°C or higher and more preferably 300°C or higher, for carbonization to produce a colored substance, more preferably producing a black colored substance, with carbon-based coloring precursors being most preferred.

The coloring precursor, when being heated, first appears as a carbonized compound, but compositionally it contains other elements in addition to carbon, and also includes layered structures, aromatic crosslinked structures and tetrahedron carbon-containing disordered structures.

Carbon-based coloring precursors are not particularly restricted, and for example, they include tar or pitch such as petroleum tar, petroleum pitch, coal tar and coal pitch, coke, polymers obtained from acrylonitrile-containing monomers, ferrocene compounds (ferrocene and ferrocene derivatives), and the like. Of these, polymers obtained from acrylonitrile-containing monomers and/or ferrocene compounds are preferred, with polyacrylonitrile being preferred as a polymer obtained from an acrylonitrile-containing monomer.

Moreover, in a not claimed incident examples of the porous polyimide film which may be used for the preset invention also include a porous polyimide film which can be obtained by molding a polyamic acid solution derived from a tetracarboxylic acid component and a diamine component followed by heat treatment without using the coloring precursor.

The porous polyimide film produced without using the coloring precursor may be produced, for example, by casting a polyamic acid solution into a film, the polyamic acid solution being composed of 3 to 60% by mass of polyamic acid having an intrinsic viscosity number of 1.0 to 3.0 and 40 to 97% by mass of an organic polar solvent, immersing or contacting in a coagulating solvent containing water as an essential component, and imidating the porous film of the polyamic acid by heat treatment. In this method, the coagulating solvent containing water as an essential component may be water, or a mixed solution containing 5% by mass or more and less than 100% by mass of water and more than 0% by mass and 95% by mass or less of an organic polar solvent. Further, after the imidation, one surface of the resulting porous polyimide film may be subjected to plasma treatment.

The tetracarboxylic dianhydride which may be used for the production of the porous polyimide film may be any tetracarboxylic dianhydride, selected as appropriate according to the properties desired. Specific examples of tetracarboxylic dianhydrides include biphenyltetracarboxylic dianhydrides such as pyromellitic dianhydride, 3,3',4,4'-biphenyltetracarboxylic dianhydride (s-BPDA) and 2,3,3',4'-biphenyltetracarboxylic dianhydride (a-BPDA), oxydiphthalic dianhydride, diphenylsulfone-3,4,3',4'-tetracarboxylic dianhydride, bis(3,4-dicarboxyphenyl)sulfide dianhydride, 2,2-bis(3,4-dicarboxyphenyl)-1,1,1,3,3,3-hexafluoropropane dianhydride, 2,3,3',4'-benzophenonetetracarboxylic dianhydride, 3,3',4,4'-benzophenonetetracarboxylic dianhydride, bis(3,4-dicarboxyphenyl)methane dianhydride, 2,2-bis(3,4-dicarboxyphenyl)propane dianhydride, p-phenylenebis(trimellitic acid monoester acid anhydride), p-biphenylenebis(trimellitic acid monoester acid anhydride), m-terphenyl-3,4,3',4'-tetracarboxylic dianhydride, p-terphenyl-3,4,3',4'-tetracarboxylic dianhydride, 1,3-bis(3,4-dicarboxyphenoxy)benzene dianhydride, 1,4-bis(3,4-dicarboxyphenoxy)benzene dianhydride, 1,4-bis(3,4-dicarboxyphenoxy)biphenyl dianhydride, 2,2-bis[(3,4-dicarboxyphenoxy)phenyl]propane dianhydride, 2,3,6,7-naphthalenetetracarboxylic dianhydride, 1,4,5,8-naphthalenetetracarboxylic dianhydride, 4,4'-(2,2-hexafluoroisopropylidene)diphthalic dianhydride, and the like. Also preferably used is an aromatic tetracarboxylic acid such as 2,3,3',4'-diphenylsulfonetetracarboxylic acid. These may be used alone or in appropriate combinations of two or more.

Particularly preferred among these are at least one type of aromatic tetracarboxylic dianhydride selected from the group consisting of biphenyltetracarboxylic dianhydride and pyromellitic dianhydride. As a biphenyltetracarboxylic dianhydride there may be suitably used 3,3',4,4'-biphenyltetracarboxylic dianhydride.

As diamine which may be used for the production of the porous polyimide film, any diamine may be used. Specific examples of diamines include the following.

1) Benzenediamines with one benzene nucleus, such as 1,4-diaminobenzene(paraphenylenediamine), 1,3-diaminobenzene, 2,4-diaminotoluene and 2,6-diaminotoluene;

2) diamines with two benzene nuclei, including diaminodiphenyl ethers such as 4,4'-diaminodiphenyl ether and 3,4'-diaminodiphenyl ether, and 4,4'-diaminodiphenylmethane, 3,3'-dimethyl-4,4'-diaminobiphenyl, 2,2'-dimethyl-4,4'-diaminobiphenyl, 2,2'-bis(trifluoromethyl)-4,4'-diaminobiphenyl, 3,3'-dimethyl-4,4'-diaminodiphenylmethane, 3,3'-dicarboxy-4,4'-diaminodiphenylmethane, 3,3',5,5'-tetramethyl-4,4'-diaminodiphenylmethane, bis(4-aminophenyl)sulfide, 4,4'-diaminobenzanilide, 3,3'-dichlorobenzidine, 3,3'-dimethylbenzidine, 2,2'-dimethylbenzidine, 3,3'-dimethoxybenzidine, 2,2'-dimethoxybenzidine, 3,3'-diaminodiphenyl ether, 3,4'-diaminodiphenyl ether, 4,4'-diaminodiphenyl ether, 3,3'-diaminodiphenyl sulfide, 3,4'-diaminodiphenyl sulfide, 4,4'-diaminodiphenyl sulfide, 3,3'-diaminodiphenylsulfone, 3,4'-diaminodiphenylsulfone, 4,4'-diaminodiphenylsulfone, 3,3'-diaminobenzophenone, 3,3'-diamino-4,4'-dichlorobenzophenone, 3,3'-diamino-4,4'-dimethoxybenzophenone, 3,3'-diaminodiphenylmethane, 3,4'-diaminodiphenylmethane, 4,4'-diaminodiphenylmethane, 2,2-bis(3-aminophenyl)propane, 2,2-bis(4-aminophenyl)propane, 2,2-bis(3-aminophenyl)-1,1,1,3,3,3-hexafluoropropane, 2,2-bis(4-aminophenyl)-1,1,1,3,3,3-hexafluoropropane, 3,3'-diaminodiphenyl sulfoxide, 3,4'-diaminodiphenyl sulfoxide and 4,4'-diaminodiphenyl sulfoxide;

3) diamines with three benzene nuclei, including 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene, 1,4-bis(3-aminophenoxy)benzene, 1,4-bis(4-aminophenoxy)benzene, 1,3-bis(3-aminophenoxy)-4-trifluoromethylbenzene, 3,3'-diamino-4-(4-phenyl)phenoxybenzophenone, 3,3'-diamino-4,4'-di(4-phenylphenoxy)benzophenone, 1,3-bis(3-aminophenyl sulfide)benzene, 1,3-bis(4-aminophenyl sulfide)benzene, 1,4-bis(4-aminophenyl sulfide)benzene, 1,3-bis(3-aminophenylsulfone)benzene, 1,3-bis(4-aminophenylsulfone)benzene, 1,4-bis(4-aminophenylsulfone)benzene, 1,3-bis[2-(4-aminophenyl)isopropyl]benzene, 1,4-bis[2-(3-aminophenyl)isopropyl]benzene and 1,4-bis[2-(4-aminophenyl)isopropyl]benzene;

4) diamines with four benzene nuclei, including 3,3'-bis(3-aminophenoxy)biphenyl, 3,3'-bis(4-aminophenoxy)biphenyl, 4,4'-bis(3-aminophenoxy)biphenyl, 4,4'-bis(4-aminophenoxy)biphenyl, bis[3 -(3 -aminophenoxy)phenyl] ether, bis[3-(4-aminophenoxy)phenyl]ether, bis[4-(3-aminophenoxy)phenyl]ether, bis[4-(4-aminophenoxy)phenyl] ether, bis[3-(3-aminophenoxy)phenyl]ketone, bis[3 -(4-aminophenoxy)phenyl]ketone, bis[4-(3-aminophenoxy)phenyl]ketone, bis[4-(4-aminophenoxy)phenyl]ketone, bis[3-(3-aminophenoxy)phenyl] sulfide, bis[3-(4-aminophenoxy)phenyl] sulfide, bis[4-(3-aminophenoxy)phenyl] sulfide, bis[4-(4-aminophenoxy)phenyl] sulfide, bis[3-(3-aminophenoxy)phenyl]sulfone, bis[3-(4-aminophenoxy)phenyl]sulfone, bis[4-(3-aminophenoxy)phenyl]sulfone, bis[4-(4-aminophenoxy)phenyl]sulfone, bis[3-(3-aminophenoxy)phenyl]methane, bis[3-(4-aminophenoxy)phenyl]methane, bis[4-(3-aminophenoxy)phenyl]methane, bis[4-(4-aminophenoxy)phenyl]methane, 2,2-bis[3-(3-aminophenoxy)phenyl]propane, 2,2-bis[3-(4-aminophenoxy)phenyl]propane, 2,2-bis[4-(3-aminophenoxy)phenyl]propane, 2,2-bis[4-(4-aminophenoxy)phenyl]propane, 2,2-bis[3-(3-aminophenoxy)phenyl]-1,1,1,3,3,3-hexafluoropropane, 2,2-bis[3-(4-aminophenoxy)phenyl]-1,1,1,3,3,3-hexafluoropropane, 2,2-bis[4-(3-aminophenoxy)phenyl]-1,1,1,3,3,3-hexafluoropropane and 2,2-bis[4-(4-aminophenoxy)phenyl]-1,1,1,3,3,3-hexafluoropropane.

These may be used alone or in mixtures of two or more. The diamine used may be appropriately selected according to the properties desired.

Preferred among these are aromatic diamine compounds, with 3,3'-diaminodiphenyl ether, 3,4'-diaminodiphenyl ether, 4,4'-diaminodiphenyl ether, paraphenylenediamine, 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene and 1,4-bis(3-aminophenoxy)benzene being preferred for use. Particularly preferred is at least one type of diamine selected from the group consisting of benzenediamines, diaminodiphenyl ethers and bis(aminophenoxy)phenyl.

From the viewpoint of heat resistance and dimensional stability under high temperature, the porous polyimide film which may be used for the invention is preferably formed from a polyimide obtained by combination of a tetracarboxylic dianhydride and a diamine, having a glass transition temperature of 240°C or higher, or without a distinct transition point at 300°C or higher.

From the viewpoint of heat resistance and dimensional stability under high temperature, the porous polyimide film which may be used for the invention is preferably a porous polyimide film comprising one of the following aromatic polyimides.

(i) An aromatic polyimide comprising at least one tetracarboxylic acid unit selected from the group consisting of biphenyltetracarboxylic acid units and pyromellitic acid units, and an aromatic diamine unit,

(ii) an aromatic polyimide comprising a tetracarboxylic acid unit and at least one type of aromatic diamine unit selected from the group consisting of benzenediamine units, diaminodiphenyl ether units and bis(aminophenoxy)phenyl units,

and/or,

(iii) an aromatic polyimide comprising at least one type of tetracarboxylic acid unit selected from the group consisting of biphenyltetracarboxylic acid units and pyromellitic acid units, and at least one type of aromatic diamine unit selected from the group consisting of benzenediamine units, diaminodiphenyl ether units and bis(aminophenoxy)phenyl units.

The porous polyimide film used in the present invention is preferably a three-layer structure porous polyimide film having a surface layer A and a surface layer B, the surface layers having a plurality of pores, and a macrovoid layer sandwiched between the surface layers A and B; wherein an average pore diameter of the pores present in the surface layer A is 0.01 $\mu$m to 15 $\mu$m, and the mean pore diameter present on the surface layer B is 20 $\mu$m to 100 $\mu$m; wherein the macrovoid layer has a partition wall bonded to the surface layers A and B, and a plurality of macrovoids surrounded by such a partition wall and the surface layers A and B; wherein the thickness of the macrovoid layer, and the surface layers A and B is 0.01 to 20 $\mu$m, wherein the pores on the surface layers A and B communicate with the macrovoid, the total film thickness is 5 to 500 $\mu$m, and the porosity is 40% or more and less than 95%. In this case, at least one partition wall in the macrovoid layer has one or two or more pores connecting the neighboring macrovoids and having the average pore diameter of 0.01 to 100 $\mu$m, preferably 0.01 to 50 $\mu$m.

For example, porous polyimide films described in WO2010/038873, Japanese Unexamined Patent Publication (Kokai) No. 2011-219585 or Japanese Unexamined Patent Publication (Kokai) No. 2011-219586 may be used for the present invention.

1-2. Porous polyethersulfone film (Porous PES film)

The porous polyethersulfone film which may be used for the present invention contains polyethersulfone and typically consists substantially of polyethersulfone. Polyethersulfone may be synthesized by the method known to those skilled in the art. For example, it may be produced by a method wherein a dihydric phenol, an alkaline metal compound and a dihalogenodiphenyl compound are subjected to polycondensation reaction in an organic polar solvent, a method wherein an alkaline metal di-salt of a dihydric phenol previously synthesized is subjected to polycondensation reaction dihalogenodiphenyl compound in an organic polar solvent or the like.

Examples of an alkaline metal compound include alkaline metal carbonate, alkaline metal hydroxide, alkaline metal hydride, alkaline metal alkoxide and the like. Particularly, sodium carbonate and potassium carbonate are preferred.

Examples of a dihydric phenol compound include hydroquinone, catechol, resorcin, 4,4'-biphenol, bis (hydroxyphenyl)alkanes (such as 2,2-bis(hydroxyphenyl)propane, and 2,2-bis(hydroxyphenyl)methane), dihydroxydiphenylsulfones, dihydroxydiphenyl ethers, or those mentioned above having at least one hydrogen on the benzene rings thereof substituted with a lower alkyl group such as a methyl group, an ethyl group, or a propyl group, or with a lower alkoxy group such as a methoxy group, or an ethoxy group. As the dihydric phenol compound, two or more of the aforementioned compounds may be mixed and used.

Polyethersulfone may be a commercially available product. Examples of a commercially available product include SUMIKAEXCEL 7600P, SUMIKAEXCEL 5900P (both manufactured by Sumitomo Chemical Company, Limited).

The logarithmic viscosity of the polyethersulfone is preferably 0.5 or more, more preferably 0.55 or more from the viewpoint of favorable formation of a macrovoid of the porous polyethersulfone film; and it is preferably 1.0 or less, more preferably 0.9 or less, further preferably 0.8 or less, particularly preferably 0.75 or less from the viewpoint of the easy production of a porous polyethersulfone film.

Further, from the viewpoints of heat resistance and dimensional stability under high temperature, it is preferred that the porous polyether sulfone film or polyethersulfone as a raw material thereof has a glass transition temperature of 200°C or higher, or that a distinct glass transition temperature is not observed.

The method for producing the porous polyether sulfone film which may be used for the present invention is not particularly limited. For example, the film may be produced by a method including the following steps:

a step in which polyethersulfone solution containing 0.3 to 60% by mass of polyethersulfone having logarithmic viscosity of 0.5 to 1.0 and 40 to 99.7% by mass of an organic polar solvent is casted into a film, immersed in or contacted with a coagulating solvent containing a poor solvent or non-solvent of polyethersulfone to produce a coagulated film having pores; and

a step in which the coagulated film having pores obtained in the above-mentioned step is heat-treated for coarsening of the aforementioned pores to obtain a porous polyether sulfone film;

wherein the heat treatment includes the temperature of the coagulated film having the pores is raised higher than the glass transition temperature of the polyethersulfone, or up to 240°C or higher.

The porous polyether sulfone film which can be used in the present invention is preferably a porous polyether sulfone film having a surface layer A, a surface layer B, and a macrovoid layer sandwiched between the surface layers A and B,

wherein the macrovoid layer has a partition wall bonded to the surface layers A and B, and a plurality of macrovoids surrounded by such a partition wall and the surface layers A and B, the macrovoids having the average pore diameter in the planar direction of the film of 10 to 500 $\mu$m;
wherein the thickness of the macrovoid layer is 0.1 to 50 $\mu$m,
each of the surface layers A and B has a thickness of 0.1 to 50 $\mu$m,
wherein one of the surface layers A and B has a plurality of pores having the average pore diameter of more than 5 $\mu$m and 200 $\mu$m or less, while the other has a plurality of pores having the average pore diameter of 0.01 $\mu$m or more and less than 200 $\mu$m,
wherein one of the surface layers A and B has a surface aperture ratio of 15% or more while other has a surface aperture ratio of 10% or more,
wherein the pores of the surface layers A and B communicate with the macrovoids,
wherein the porous polyether sulfone film has total film thickness of 5 to 500 $\mu$m and a porosity of 50 to 95%.

As the aforementioned porous polymer film used as a cell culture support in the cell culture device of the present invention has pores with slightly hydrophilic properties, stable liquid retention is achieved within the porous polymer film and a moist environment that is resistant to drying is maintained. Thus, survival and proliferation of cells can be achieved even with very little medium compared to conventional cell culture devices that use a cell culture support. Furthermore, as culturing is possible even if a part of or the entire porous polymer film is exposed to air, oxygen can be efficiently supplied to the cells and a large number of cells can be cultured.

According to the present invention, very little medium is used, and as the porous polymer film used as the culture support can be exposed to a gaseous phase, the supply of oxygen to the cells can be sufficiently performed through diffusion. Thus, there is no particular need for an oxygen supply device in the present invention.

2. Cell culture device

The present invention relates to a cell culture device having a siphon configuration or a "shishi odoshi" configuration and comprises a porous polymer film, a cell culture part that accommodates the porous polymer film, a liquid storage part in which the cell culture part is stored, a medium supply means disposed above the liquid storage part, and a medium reservoir part provided with a medium discharge means and disposed below the liquid storage part characterized in that the medium temporarily stored in the liquid storage part is discharged intermittently into the medium reservoir part. The cell culture device according to the present invention is typically exemplified using a siphon-type cell culture device and a "shishi odoshi"-type cell culture device. However, as described above, provided the cell culture device is characterized by intermittently discharging medium temporarily stored in a liquid storage part into a medium reservoir part, there is no need for such classifications. These cell culture devices are generally referred to herein as "the cell culture device of the present invention". The following embodiments of the cell culture device of the present invention will be described with reference to the drawings.

(1) Siphon-type cell culture device

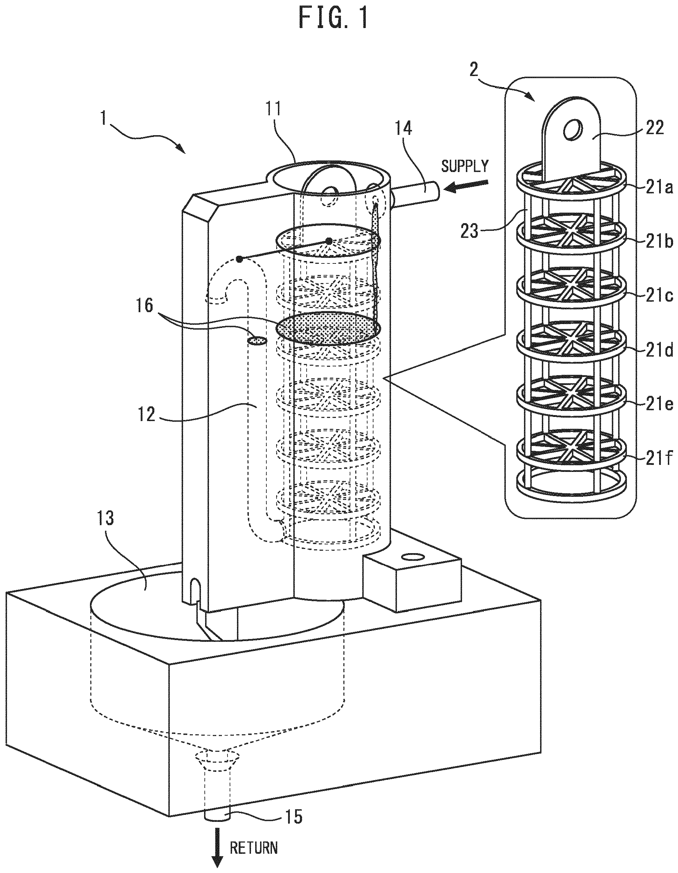

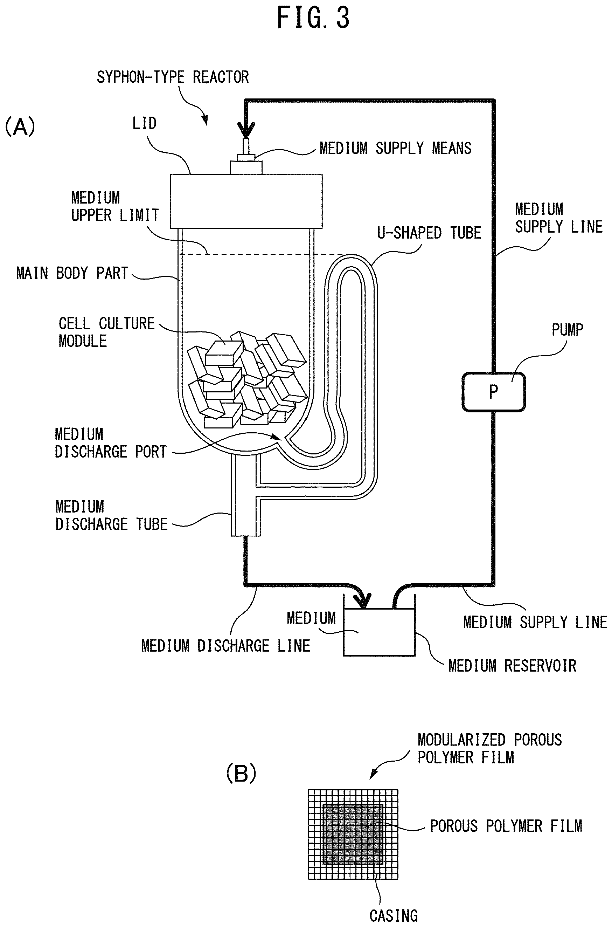

One embodiment of the present invention relates to a cell culture device comprising: a porous polymer film; a cell culture part that accommodates the porous polymer film; a liquid storage part in which the cell culture part is stored; a medium supply means disposed on an upper part of the liquid storage part; an inverted U-shaped tube communicating with a bottom part of the liquid storage part; a medium recovery means provided below the other end of the inverted U-shaped tube; and a medium discharge means disposed on the medium recovery means; wherein the porous polymer film is a three-layer structured porous polymer film having a surface layer A and a surface layer B having a plurality of pores, and a macrovoid layer provided between the surface layer A and the surface layer B, the average pore diameter of the pores present in the surface layer A is smaller than the average pore diameter of the pores present in the surface layer B, and the macrovoid layer has a partition wall that is bound to the surface layers A and B, and a has plurality of macrovoids enclosed by the partition wall and the surface layers A and B; and is characterized in that when the liquid surface 16 (FIG. 1) of the medium supplied to the liquid storage part from the medium supply means reaches a top part of the inverted U-shaped tube, the medium is intermittently discharged into the medium recovery means due to the siphon principle. In this device it is possible to use a cell culture module (e.g., FIG. 3(B)) in place of the porous polymer film.

FIG. 1 is a diagram showing a typical example of the siphon-type cell culture device of the present invention. A cell culture part support body 2, in which one or more cell culture parts 21a to 21f are held by struts 23 in the vertical direction, can be accommodated in a liquid storage part 11 and is further provided with a handle 22 so that after culturing is completed the cell culture support body 2 can be removed therefrom. The shape of a cross section of the cell culture part support body 2 can be appropriately adjusted so as to correspond to the liquid storage part 11. The shape of a cross section of the liquid storage part 11 is not particularly limited and may be a square, rectangle, circle, or triangle but is preferably a circle. The shape of the liquid storage part 11 and the cell culture part support body 2 may be appropriately changed according to the purpose.

The cell culture parts 21a to 21f have a perforated structure allowing the flow of medium therethrough and a porous polymer film (not shown) can be installed or accommodated therein. The number of stages of the cell culture part is not particularly limited. However, after initiating storage of the medium in the liquid storage part 11, there is a time lag from when the medium starts to soak the lowest stage cell culture part 21f to when the medium soaks the highest stage cell culture part 21a, and thus there are preferably 100 stages or less, more preferably 50 stages or less, even more preferably 30 stages or less, and still more preferably 10 stages or less, and there may be for example 9, 8, 7, 6, 5, 4, 3, 2, or 1 stage. Furthermore, in a cell culture device, in order to set the time cells cultured by each cell culture part are exposed to a gaseous phase to a constant amount, it is preferable for the amount of medium supplied per unit time to the liquid storage part 11 from the medium supply means 14 to be equal to the amount of medium discharged per unit time from the other end of the inverted U-shaped tube 12 provided at the liquid storage part.

The liquid storage part 11 is provided with a medium supply means 14 on an upper part thereof and an inverted U-shaped tube 12 on a bottom part thereof. The medium supply means 14 is a means for supplying the medium stored in a medium storage tank (not shown) or medium recovery means 13 to the liquid storage part 11 and may be provided with a regulating part that regulates the flow rate of the medium. Furthermore, the medium supply means to the liquid storage part 11, which is not shown, may be a mist supply nozzle that can supply the culture liquid as microdroplets. In the present invention, provided the mist supply nozzle is a means that supplies a medium as droplets, there is no limit to the number of nozzles, the arrangement thereof, or the size of the droplets supplied thereby.

In one embodiment, the cell culture device may be provided with a medium discharge line in communication with a medium discharge means 15 at one end and a medium supply line in communication with a medium supply means 14 at one end. The other end of the medium discharge line is in communication with the other end of the medium supply line via a pump and the medium can be circulated. The type of pump for pumping up the culture liquid is not particularly limited but, for example, a peristaltic pump with which the flow rate can be regulated can be used. The aforementioned regulation part can be used instead of the pump.

The medium recovery means 13 has the role of temporarily storing medium that was stored in the liquid storage part and discharged therefrom and thus it is preferable if the capacity thereof is the same as or exceeds that of the liquid storage part 11.

(2) "Shishi odoshi"-type cell culture device

One embodiment of the present invention relates to a cell culture device comprising: a porous polymer film; a box-shaped body, one end of which is a liquid storage part, the other end of which is a bottom part, which is provided with a cell culture part in the liquid storage part that accommodates the porous polymer film; a rotary shaft provided in a part at the bottom surface side of the box-shaped body; a bearing supporting the rotary shaft; a medium supply means disposed above the liquid storage part; and a medium recovery means disposed below the liquid storage part; wherein the porous polymer film is a three-layer structured porous polymer film having a surface layer A and a surface layer B having a plurality of pores, and a macrovoid layer provided between the surface layer A and the surface layer B, the average pore diameter of the pores present in the surface layer A being smaller than the average pore diameter of the pores present in the surface layer B, and the macrovoid layer having a partition wall that is bound to the surface layers A and B, and having a plurality of macrovoids enclosed by the partition wall and the surface layers A and B; and is characterized in that when the medium supplied to the liquid storage part from the medium supply means exceeds the balance of weight of the bottom part of the box-shaped body, the medium from the liquid storage part is intermittently discharged into the medium recovery means and once the medium is discharged, the box-shaped body returns to the original horizontal position.

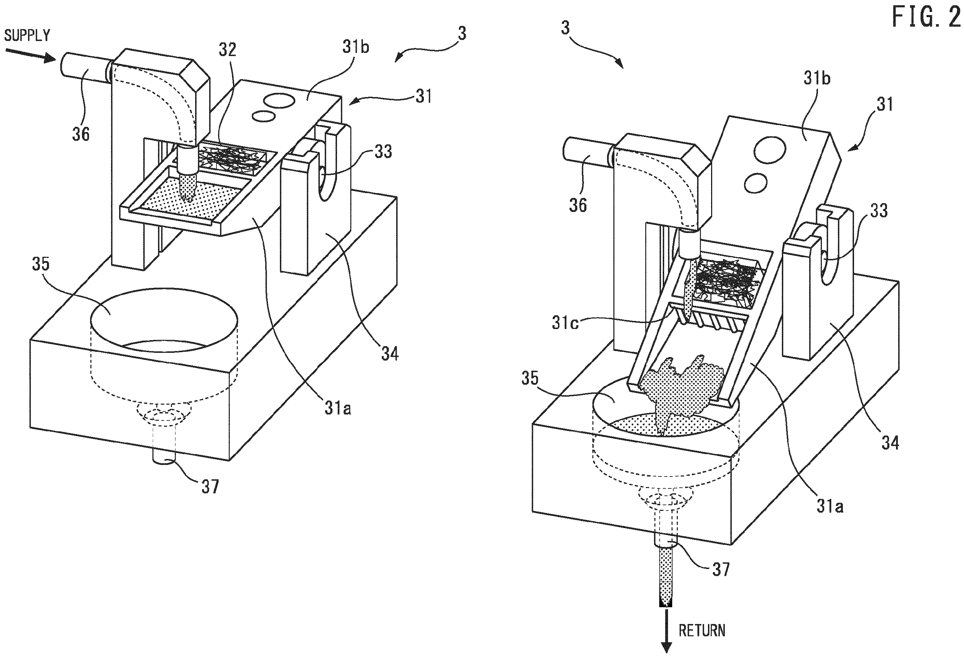

FIG. 2 is a diagram showing a typical example of the "shishi odoshi"-type cell culture device of the present invention. The "shishi odoshi"-type cell culture device has a function in common with the siphon-type cell culture device in that once the medium is stored in the liquid storage part, the medium is discharged therefrom after a fixed period. The box-shaped body 3 is provided with a liquid storage part 31a and a bottom part 31b. The bottom part 31b has a weight such that when the weight of a medium supplied to the liquid storage part 31a exceeds a threshold value and the box-shaped body 3 begins to incline, the bottom part can return to the horizontal position once the medium has been discharged. The liquid storage part 31a has the role of accommodating the porous polymer film, and, in order to prevent the porous polymer film from falling out when the box-shaped body 3 is inclined, a partition plate 31c having holes, or a fence-like or mesh-like structure that allows the flow of liquid therethrough can be provided. Note that in order for the medium supplied from the medium supply means to avoid directly hitting the porous polymer film, the arrangement of the medium supply means can be appropriately changed so that the medium can be supplied to a side where the porous polymer film is not located by partitioning with the partition plate.

Furthermore, the bottom side of box-shaped body 3 may be provided with a rotary shaft 33 in one part of the bottom surface side and may be provided with a support means (not shown) in one part of the bottom surface side of the bottom part 31b, favorably, to keep the box-shaped body 3 in a horizontal state from when the liquid storage part 31a is empty until the weight of the medium reaches the threshold value, as necessary. The rotary shaft 33 is supported at both ends thereof by bearings 34 and facilitates the toppling motion of the box-shaped body 3. However, the means for allowing the toppling motion of the box-shaped body is not limited thereto and may be a configuration that allows the toppling motion around a tabular toppling shaft attached to a part of the bottom surface side as a toppling fulcrum. A person skilled in the art could confirm that a medium supply means, medium recovery means, medium discharge means, each type of line, and pump that constitute the siphon-type cell culture device can be appropriately used therefor.

The porous polymer film used in an embodiment of the present invention may, for example, be: i) folded up; ii) wound into a roll shape; iii) connected to a sheet or small piece by a filamentous structure; and/or iv) tied in a rope shape; and is provided in the planar flow path. Further, the porous polymer film used in the present embodiment may be v) laminated in two or more layers and provided in the planar flow path. By processing the porous polymer films into forms like i) to v), many porous polymer films can be added to a fixed quantity of cell culture medium.

The porous polymer film used in an embodiment of the present invention, may be a modularized porous polymer film (referred to as "modularized porous polymer film" below). The "modularized porous polymer film" herein is a porous polymer film accommodated in a casing. The phrase "a cell culture module" may be expressed simply as "a module", both expression can be used interchangeably to indicate the same meaning.

The casing provided with a modularized porous polymer film used in an embodiment of the present invention has two or more cell medium flow ports, and the medium flows into or out of the casing through the cell culture medium flow ports. The diameter of the medium flow inlet of the casing is preferably larger than the diameter of the cell so as to enable cell to flow into the casing. In addition, the diameter of the medium flow inlet is preferably smaller than the diameter through which the porous polymer film flows out from the medium flow inlet. The diameter smaller than the diameter through which the porous polymer film flows out may be appropriately selected depending on the shape and size of the porous polymer film contained in the casing. For example, when the porous polymer film has string-like shape, the diameter is not particularly limited so long as it is smaller than the width of the shorter side of the porous polymer film so that the porous polymer film is prevented from flowing out. It is preferred to provide as many medium flow inlets as possible so that the cell culture medium may be easily supplied into and/or discharged from the casing. It is preferably 5 or more, preferably 10 or more, preferably 20 or more, preferably 50 or more, and preferably 100 or more. As for the medium flow inlet, the casing may have a mesh-like structure in part or as a whole. Moreover, the casing itself may be mesh-like. In the present invention, examples of mesh-like structure include, but not limited to, those including longitudinal, transverse, and/or oblique elements wherein individual apertures form medium flow inlets which allow the fluid to pass therethrough.

The casing for the modularized porous polymer film used in an embodiment of the present invention may be polystyrene, polycarbonate, polymethylmethacrylate, polyethyleneterephthalate, and metals such as stainless steel (also referred to as "steel"). However, provided the culture of cells is not affected, there are no particular restrictions on the material.

The cell culture module is contained within the casing with:

(i) the two or more independent porous polymer films being aggregated;
(ii) the porous polymer films being folded up;
(iii) the porous polymer films being wound into a roll-like shape; and/or
(iv) the porous polymer film being tied together into a rope-like shape.

In this specification, "two or more independent porous polymer films are aggregated and contained within a casing" means that two or more independent porous polymer films are aggregated and contained in a predetermined space surrounded by a casing. The two or more independent porous polymer films may be immovably fixed by fixing at least one point of the porous polymer film to at least one point of the casing by an arbitrary method. In addition, the two or more independent porous polymer films may be fragments. The fragments may take any shape such as a circle, an ellipse, a square, a triangle, a polygon, a string, etc., but a substantially square shape is preferred. The fragments may be any size. When it has a substantially square shape, the side may be any length, but, for example, preferably 80 mm or less, preferably 50 mm or less, more preferably 30 mm or less, still more preferably 20 mm or less, and may be 10 mm or less. In addition, when the fragments of the porous polymer film are substantially square, it may be formed so that length of each side may match the inner wall or may be shorter than each side of the inner wall (e.g. shorter by about 0.1 mm to 1 mm), rendering the porous polymer film immovable in the casing. This can protect cells growing in the porous polymer film from stress to be applied, resulting in suppression of apoptosis or the like and enabling a stable cell culture in a large amount. The string-like porous polymer film may be contained within the casing, with: (ii) the porous polymer films being folded up; (iii) the porous polymer films being wound into a roll-like shape; and/or (iv) the porous polymer film being tied together into a rope-like shape, as described below. In addition, any number of the porous polymer films may be stacked to aggregate and contain the 2 or more independent porous polymer films in the casing. In this case, a liner may be provided between the porous polymer films. Providing a liner may enable efficient supply of a medium between the stacked porous polymer films. The liner may be not particularly limited so long as it may have function to form an arbitrary space between the stacked porous polymer films to efficiently supply medium. For example, a planar construct having a mesh structure may be used. As for material of the liner, for example, a mesh made of polystyrene, polycarbonate, polymethyl methacrylate, polyethylene terephthalate, stainless steel or the like may be used without limitation. When there is a liner having a mesh structure, the material may be appropriately selected so long as the mesh may have openings such that a medium may be supplied between the stacked porous polymer films.

In this specification, "the porous polymer films being folded up" means a porous polymer film which is folded up in the casing, and thus it is rendered immovable in the casing by frictional force between each surfaces of the porous polymer film and/or the inner surface of the casing. In this specification, "being folded" may indicate the porous polymer film being creased or creaseless.

In this specification, "the porous polymer films being wound into a roll-like shape" means the porous polymer film being wound into a roll-like shape and thus it is rendered immovable in the casing by frictional force between each surfaces of the porous polymer film and/or the inner surface of the casing. Moreover, the porous polymer film being tied together into a rope-like shape means, for example, more than one porous polymer films in rectangle strip shape are knitted into a rope-shape by arbitrary method, rendering the porous polymer films immovable by the mutual frictional force of the porous polymer films. It is also possible that (i) the two or more independent porous polymer films being aggregated; (ii) the porous polymer films being folded up; (iii) the porous polymer films being wound into a roll-like shape; and/or (iv) the porous polymer film being tied together into a rope-like shape may be combined and contained within a casing.

In this specification, "the porous polymer film being immovable in the casing" means that the porous polymer film is contained in the casing so that the porous polymer film is continually morphologically unchanged during culturing the cell culture module in the cell culture medium. In other words, the porous polymer film itself is continually prevented from waving by fluid. Since the porous polymer film is kept immovable in the casing, the cell growing in the porous polymer film is protected from stress to be applied, enabling stable cell culture without cells being killed.

(3) Dry heat sterilized syphon-type cell cultured device

A cell culture device can be provided where the entirety of siphon-type cell culture device defined in the aforementioned (1) can be completely dry sterilized. A dry sterilized siphon-type cell culture device is provided (FIG. 3(A)) in which a glass heat resistant siphon reactor is used and into which is incorporated a metal module (FIG. 3(B)) made from stainless steel mesh which is accommodated in the reactor and which has a porous polyamide film therein. As long as the function of intermittently discharging medium by the siphon principle is provided, there are no restrictions on the glass heat resistant siphon reactor, which may for example, be based on a Soxhlet extractor.

3. Cell culture method using cell culture device

<Step of applying cells to porous polymer film>

The specific step by which the cells used in the present invention are applied to the porous polymer film is not particularly limited. The process described herein or any suitable means for applying cells to a film-like support may be adopted. The means for applying the cells to the porous polymer film in the method of the present invention include the following embodiments but are not limited thereby.

(A) A not claimed incident including the step of inoculating cells on the surface of a porous polymer film.
(B) A not claimed incident including the step of placing a cell suspension onto the surface of a dried porous polymer film which is either left standing, moved to promote the outflow of liquid, or a part of the surface thereof is stimulated so that the cell suspension is absorbed into the film such that the cells in the cell suspension are retained in the film and the moisture flows out.
(C) A not claimed incident including the step of moistening one or both surfaces of the porous polymer film with the cell culture liquid or a sterilized liquid and loading the moistened porous polymer film with the cell suspension such that the cells in the cell suspension are retained in the film and the moisture flows out.

Incident (A) includes the direct inoculation of cells or a cell cluster onto the surface of the porous polymer film. the porous polymer film is placed in the cell suspension such that the cell culture liquid permeates the film surface.

The cells inoculated onto the surface of the porous polymer film adhere to the porous polymer film and permeate into the pores. The cells preferably adhere to the porous polymer film without the addition of any particular external physical or chemical forces. The cells inoculated onto the surface of the porous polymer film can stably grow and proliferate in and/or on the surface of the film. A variety of different forms can be achieved according to the position of the film on which the cells grow and proliferate.

In incident (B) a cell suspension is placed on the surface of a dried porous polymer film. In order for the cell suspension to permeate into the film, the porous polymer film is left standing, moved to promote the outflow of liquid, or a part of the surface thereof is stimulated so that the cell suspension is absorbed into the film. Although not bound by theory, it is considered that this permeation is dependent on a property derived from the shape, etc., of each surface of the porous polymer film. Cells are absorbed into and inoculated in the part of the film loaded with the cell suspension.

Alternatively, like incident (C), a part or the whole of one or both surfaces of the porous polymer film may be moistened with cell culture liquid or a sterilized liquid then the cell suspension loaded onto the moistened porous polymer film. In such cases, the permeation rate of the cell suspension substantially increases.

For example, a method of moistening an extremely small part of the film (hereinafter referred to as "one-point wetting method") focusing on the prevention of splashing from the film can be used. The one-point wetting method is practically very similar to the dry method incident (B)) in which the film is not moistened. However, regarding the small portion that is moistened, it is considered that the passage of the cell solution through the film becomes rapid. Furthermore, a method in which a cell suspension is loaded onto a porous polymer film that is sufficiently moistened over the entirety of one or both surfaces (hereinafter referred to as "wet film") can also be used (hereinafter referred to as "wet film method"). In such cases, the permeation rate of the cell suspension through the entire porous polymer film substantially increases.

In incidents (B) and (C), cells in the cell suspension are retained in the film and moisture flows out. The concentration of cells in the cell suspension is thereby increased, and treatment involving, for example, the outflow of unnecessary components that do not include the cells together with the moisture is also possible.

There are cases when incident (A) is referred to as "natural inoculation" and incidents (B) and (C) are referred to as "absorption inoculation".

It is preferable for living cells to be selectively retained in the porous polymer film although this is in no ways limiting. Thus, in a method of a preferable embodiment of the present invention, living cells are retained in the porous polymer film, and dead cells preferentially flow out with the moisture.

The sterilized liquid used in embodiment (C) is not particularly limited but is a sterilized buffer solution or sterilized water. The buffer solution is, for example, (+) and (-) Dulbecco's PBS, (+) and (-) Hanks's Balanced Salt Solution. Examples of the buffer solution are indicated in Table 1 below.

**[Table 1]**

Table 1

| Component | Concentration (mmol/L) | Concentration (g/L) |
|---|---|---|
| NaCl | 137 | 8.00 |
| KCl | 2.7 | 0.20 |
| $Na_2HPO_4$ | 10 | 1.44 |
| $KH_2PO_4$ | 1.76 | 0.24 |
| pH(-) | 7.4 | 7.4 |

Furthermore, the method includes the application of the cells to the porous polymer film involves adhering the cells onto the film by making suspended adhesive cells coexist with the porous polymer film in suspension. For example, in order to apply the cells to the porous polymer film, a cell culture medium, cells and one or more porous polymer films may be added to a cell culture vessel. When the cell culture medium is liquid, the porous polymer film is suspended in the cell culture medium. Due to the properties of the porous polymer film the cells are able to adhere to the porous polymer film. Thus, even if the cells are not suited to natural suspension cultures, culturing is possible with the porous polymer film suspended in the cell culture medium. The cells are preferably adhered to the porous polymer film. "Spontaneously adhere" is defined as the retention of cells on the surface or in the interior of the porous polymer film without the addition of any particular external physical or chemical forces.

The aforementioned application of cells to the porous polymer film may include the combination of two or more methods. For example, the cells may be applied to the porous polymer film by combining two or more methods from among incidents (A) to (C). It is possible to culture cells supported on the porous polymer film by the application thereof to the planar flow paths of the aforementioned cell culture device.

In addition, a medium in which cells are suspended may be inoculated as droplets by a cell supply means onto the planar flow path on which is provided the porous polymer film in advance.

"Suspended cells" as defined herein include adherent cells that can be suspension cultured in a medium wherein the cells are obtained by, for example, forcibly suspending adherent cells in a medium by using a proteolytic enzyme such as trypsin or by using a publicly-known conditioning step.

The types of the cells which may be used for the present invention may be selected from the group consisting of animal cells, insect cells, plant cells, yeast cells and bacteria. Animal cells are largely divided into cells from animals belonging to the subphylum Vertebrata, and cells from non-vertebrates (animals other than animals belonging to the subphylum Vertebrata). There are no particular restrictions on the source of the animal cells, for the purpose of the present specification. Preferably, they are cells from an animal belonging to the subphylum Vertebrata. The subphylum Vertebrata includes the superclass Agnatha and the superclass Gnathostomata, the superclass Gnathostomata including the class Mammalia, the class Aves, the class Amphibia and the class Reptilia. Preferably, they are cells from an animal belonging to the class Mammalia, generally known as mammals. Mammals are not particularly restricted but include, preferably, mice, rats, humans, monkeys, pigs, dogs, sheep and goats.

The types of animal cells or plant cells that may be used for the invention are not particularly restricted, but are preferably selected from the group consisting of pluripotent stem cells, tissue stem cells, somatic cells and germ cells.

The term "pluripotent stem cells", in this specification, is intended as a comprehensive term for stem cells having the ability to differentiate into cells of any tissues (pluripotent differentiating power). While not restrictive, pluripotent stem cells include embryonic stem cells (ES cells), induced pluripotent stem cells (iPS cells), embryonic germ cells (EG cells) and germ stem cells (GS cells). They are preferably ES cells or iPS cells. Particularly preferred are iPS cells, which are free of ethical problems, for example. The pluripotent stem cells used may be any publicly known ones, and for example, the pluripotent stem cells described in WO2009/123349 (PCT/JP2009/057041) may be used.

The term "tissue stem cells" refers to stem cells that are cell lines capable of differentiation but only to limited specific tissues, though having the ability to differentiate into a variety of cell types (pluripotent differentiating power). For example, hematopoietic stem cells in the bone marrow are the source of blood cells, while neural stem cells differentiate into neurons. Additional types include hepatic stem cells from which the liver is formed and skin stem cells that form skin tissue. Preferably, the tissue stem cells are selected from among mesenchymal stem cells, hepatic stem cells, pancreatic stem cells, neural stem cells, skin stem cells and hematopoietic stem cells.

The term "somatic cells" refers to cells other than germ cells, among the cells composing a multicellular organism. In sexual reproduction, these are not passed on to the next generation. Preferably, the somatic cells are selected from among hepatocytes, pancreatic cells, muscle cells, bone cells, osteoblasts, osteoclasts, chondrocytes, adipocytes, skin cells, fibroblasts, pancreatic cells, renal cells and lung cells, or blood cells such as lymphocytes, erythrocytes, leukocytes, monocytes, macrophages or megakaryocytes.

The term "germ cells" refers to cells having the role of passing on genetic information to the succeeding generation in reproduction. These include, for example, gametes for sexual reproduction, i.e. the ova, egg cells, sperm, sperm cells, and spores for asexual reproduction.

The cells may also be selected from the group consisting of sarcoma cells, established cell lines and transformants. The term "sarcoma" refers to cancer occurring in non-epithelial cell-derived connective tissue cells, such as the bone, cartilage, fat, muscle or blood, and includes soft tissue sarcomas, malignant bone tumors and the like. Sarcoma cells are cells derived from sarcoma. The term "established cell line" refers to cultured cells that are maintained in vitro for long periods and reach a stabilized character and can be semi-permanently subcultured. Cell lines derived from various tissues of various species including humans exist, such as PC12 cells (from rat adrenal medulla), CHO cells (from Chinese hamster ovary), HEK293 cells (from human embryonic kidney), HL-60 cells (from human leukocytes) and HeLa cells (from human cervical cancer), Vero cells (from African green monkey kidney epithelial cells), MDCK cells (from canine renal tubular epithelial cells), HepG2 cells (from human hepatic cancer), BHK cells (newborn hamster kidney cell), NIH3T3 cells (from mouse fetal fibroblast cells). The term "transformants" refers to cells with an altered genetic nature by extracellularly introduced nucleic acid (DNA and the like).

In this specification, an "adherent cell" is generally a cell which is required to adhere itself on an appropriate surface for growth, and is also referred to as an adhesion cell or an anchorage-dependent cell. The cells intended for use in the present invention can be adherent cells more preferably cells which may be cultured even as a suspension in a medium. The adherent cells which can be suspension cultured may be obtained by conditioning the adherent cells to a state suitable for suspension culture, and include, for example, CHO cells, HEK293 cells, Vero cells, NIH3T3 cells, and cell lines derived from these cells. The cells intended for use in the present invention other than listed herein are not particularly limited so long that they may be applied to suspension culture by conditioning.

FIGs. 1, 2, and 3(A) represent a model diagram of cell culturing using a porous polymer film. These figures serve merely for illustration and the elements are not drawn to their actual dimensions. In the cell culture method of the invention, application of cells and culturing are carried out on a porous polymer film, thereby allowing culturing of large volumes of cells to be accomplished since large numbers of cells grow on the multisided connected pore sections on the inside, and the surfaces on the porous polymer film. Moreover, in the cell culture method of the invention, it is possible to culture large volumes of cells while drastically reducing the amount of medium used for cell culturing compared to the prior art. For example, large volumes of cells can be cultured even when all or a portion of the porous polymer film is not in contact with the liquid phase of the cell culture medium. In addition, the total volume of the cell culture medium in the cell culture vessel, with respect to the total porous polymer film volume including the cell survival zone, can be significantly reduced.

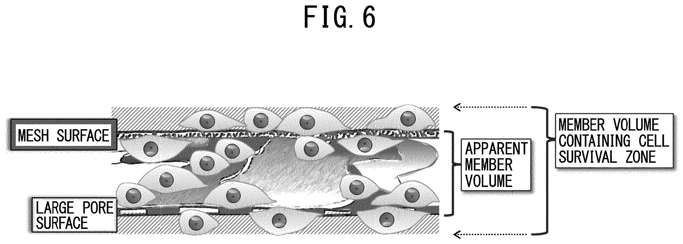

Throughout the present specification, the volume of the porous polymer film without cells, that occupies the space including the volume between the interior gaps, will be referred to as the "apparent porous polymer film volume" (see, FIG. 6). In the state where the cells are applied to the porous polymer film and the cells have been supported on the surface and the interior of the porous polymer film, the total volume of the porous polymer film, the cells and the medium that has wetted the porous polymer film interior, which is occupying the space therein, will be referred to as the "porous polymer film volume including the cell survival zone" (see, FIG. 6). When the porous polymer film has a film thickness of 25 $\mu$m, the porous polymer film volume including the cell survival zone is a value of at maximum about 50% larger than the apparent porous polymer film volume. In the method of the invention, a plurality of porous polymer films may be housed in a single cell culture vessel for culturing, in which case the total sum of the porous polymer film volume including the cell survival zone for each of the plurality of porous polymer films supporting the cells may be referred to as simply as the "total sum of the porous polymer film volume including the cell survival zone".

Using the method of the invention, cells can be satisfactorily cultured for a long period of time even under conditions in which the total volume of the cell culture medium in the cell culture vessel is 10,000 times or less of the total sum of the porous polymer film volume including the cell survival zone. Moreover, cells can be satisfactorily cultured for a long period of time even under conditions in which the total volume of the cell culture medium in the cell culture vessel is 1,000 times or less of the total sum of the porous polymer film volume including the cell survival zone. In addition, cells can be satisfactorily cultured for a long period of time even under conditions in which the total volume of the cell culture medium in the cell culture vessel is 100 times or less of the total sum of the porous polymer film volume including the cell survival zone. In addition, cells can be satisfactorily cultured for a long period of time even under conditions in which the total volume of the cell culture medium in the cell culture vessel is 10 times or less of the total sum of the porous polymer film volume including the cell survival zone.

In other words, the space (vessel) used for cell culturing can be reduced to an absolute minimum, compared to a conventional cell culture device for performing two-dimensional culture. Furthermore, when it is desired to increase the number of cells cultured, the cell culturing volume can be flexibly increased by a convenient procedure including increasing the number of layered porous polymer films. In a cell culture device comprising a porous polymer film to be used for the invention, the space (vessel) in which cells are cultured and the space (vessel) in which the cell culture medium is stored can be separate, and the necessary amount of cell culture medium can be prepared according to the number of cells to be cultured. The space (vessel) in which the cell culture medium is stored can be increased or decreased according to the purpose, or it may be a replaceable vessel, with no particular restrictions.

In the cell culture method of the invention, culturing in which the number of cells in the cell culture vessel after culturing using the porous polymer film reaches $1.0 \times 10^5$ or more, $1.0 \times 10^6$ or more, $2.0 \times 10^6$ or more, $5.0 \times 10^6$ or more, $1.0 \times 10^7$ or more, $2.0 \times 10^7$ or more, $5.0 \times 10^7$ or more, $1.0 \times 10^8$ or more, $2.0 \times 10^8$ or more, $5.0 \times 10^8$ or more, $1.0 \times 10^9$ or more, $2.0 \times 10^9$ or more, or $5.0 \times 10^9$ or more per milliliter of medium, assuming that all of the cells are evenly dispersed in the cell culture medium in the cell culture vessel, is mentioned.

It should be noted that as a method for measuring cell count during or after culture, various known methods may be used. For example, as the method for counting the number of cells in the cell culture vessel after culturing using the porous polymer film, assuming that the cells are evenly dispersed in the cell culture medium in the cell culture vessel, any publicly known method may be used. For example, a cell count method using CCK8 may be suitably used. Specifically, a Cell Counting Kit 8 (a solution reagent, commercially available from Dojindo Laboratories)(hereunder referred to as "CCK8") may be used to count the number of cells in ordinary culturing without using a porous polymer film, and the correlation coefficient between the absorbance and the actual cell count is determined. Subsequently, the cells are applied, the cultured porous polymer film may be transferred to CCK8-containing medium and stored in an incubator for 1 to 3 hours, and then the supernatant is extracted and its absorbance is 30 measured at a wavelength of 480 nm, and the cell count is determined from the previously calculated correlation coefficient.

In addition, from another point of view, for example, "mass culturing of cells" may refer to culturing in which the number of cells in the cell culture vessel after culturing using the porous polyimide film reaches $1.0 \times 10^5$ or more, $2.0 \times 10^5$ or more, $1.0 \times 10^6$ or more, $2.0 \times 10^6$ or more, $5.0 \times 10^6$ or more, $1.0 \times 10^7$ or more, $2.0 \times 10^7$ or more or $5.0 \times 10^7$ or more, $1.0 \times 10^8$ or more, $2.0 \times 10^8$ or more, or $5.0 \times 10^8$ or more, per square centimeter of porous polymer film. The number of cells contained per square centimeter of porous polymer film may be appropriately measured using a publicly known method, such as with a cell counter.

EXAMPLES

The present invention will now be explained in greater detail by Examples.
The porous polyimide films used in the following examples were prepared by forming a polyamic acid solution composition including a polyamic acid solution obtained from 3,3',4,4'-biphenyltetracarboxylic dianhydride (s-BPDA) as a tetracarboxylic acid component and 4,4'-diaminodiphenyl ether (ODA) as a diamine component, and polyacrylamide as a coloring precursor, and performing heat treatment at 250°C or higher. The resulting porous polyimide film was a three-layer structure porous polyimide film having a surface layer A and a surface layer B, the surface layers having a plurality of pores, and a macrovoid layer sandwiched between the surface layers A and B; wherein the average pore diameter of the pore present on the surface layer A was 6 $\mu$m, the average pore diameter of the pore present on the surface layer B was 46 $\mu$m, and the film thickness was 25 $\mu$m, and the porosity was 73%.

(EXAMPLE 1)

Cell culture method using porous polyimide film using a siphon-type reactor (first)

Conditioned and suspended anti-human IL-8 antibody producing CHO-DP12 cells (ATCC CRL-12445) were suspension cultured using a medium (BalanCD (trademark) CHO Growth A) until the number of viable cells per ml reached 3.9 $\times$ 106. 12 ml of the suspension culture liquid was added to one 10 cm diameter petri dish, thereafter, an outer coating (casing) was formed using a nylon mesh (30#, mesh opening 547 $\mu$m) and 12 modules, in which a fixed amount of a porous polyimide film (20 cm$^2$ per module) was added sterilely and sealed, were added to the same petri dish. After moistening the modules with the cell suspension, the petri dishes were left overnight in a $CO_2$ incubator.
The following day, the modules were removed, 10 modules were placed on the stage part of the siphon-type reactor shown in FIG. 1,350 ml of a medium (I MDM comprising FBS 2%) was stored in the liquid storage part, and the same medium was circulated at a rate of 60 ml per minute through a tube pump. Cell culturing was terminated two days later and a cell density of 5.5 $\times$ 10$^4$ cells/cm$^2$ and a total cell number of 1.2 $\times$ 10$^7$ cells were observed.

(EXAMPLE 2)

Cell culture method using porous polyimide film using a siphon-type reactor (second)

Conditioned and suspended anti-human IL-8 antibody producing CHO-DP12 cells (ATCC CRL-12445) were suspension cultured using a medium (BalanCD (trademark) CHO Growth A) until the number of viable cells per ml reached 9.9 $\times$ 10$^6$. 10 modules were put into an oxygen permeable shake culture bag and shake cultured overnight in a $CO_2$ incubator. The following day, the modules were removed from the shake culture bag, and under the same conditions as for EXAMPLE 1, cell culturing was performed in the siphon-type reactor. 350 ml of a medium (KBM 270 manufactured by Kohjin Bio) was stored in the liquid storage part, and the same medium was circulated at a rate of 60 ml per minute through a tube pump. Cell culturing was terminated four days later and a cell density of 1.0 $\times$ 10$^5$ cells/cm$^2$ and a total cell number of 2.1 $\times$ 10$^7$ cells were observed.

(EXAMPLE 3)

Metal module and glass heat resistant siphon reactor

A metal module was prepared (the embodiment of FIG. 3 (B)) comprising a stainless steel mesh casing, an inner lining, and a porous polyimide film such that a sterilization procedure could be completed by a simple total dry heat sterilization, taking complete advantage of the heat resistivity of the porous polyimide film. Specifically, the metal module was prepared by sealing, in a steel mesh casing, a laminate of 1 cm $\times$ 1 cm porous polyimide films and stainless steel mesh (referred to as "inner lining" and not shown) with the same surface area (laminated in the following order: three porous polyimide films; one inner lining; four porous polyimide films; one inner lining; and three porous polyimide films). The procedure was carried out non-sterilely in an open space.
The glass heat resistant siphon reactor for operating the metal module was designed based on a Soxhlet extractor and in order to be heat resistant was made entirely of glass and metal (FIG. 3 (A)). A glass heat resistant siphon reactor into which metal modules are placed is shown. The device was assembled by stacking 30 steel modules non-sterilely into the reactor. Thereafter, the reactor part was wrapped in aluminum foil and dry heat sterilized at 190 degrees Celsius for 80 minutes and then left to cool.

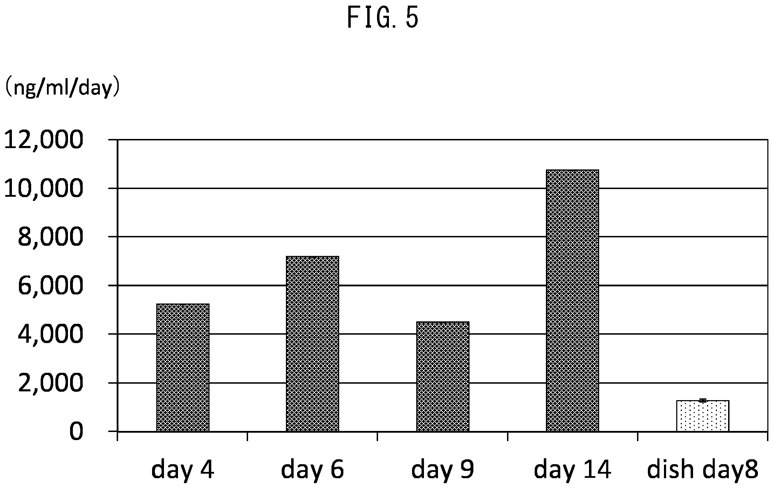

Next, dry culturing of human dermal fibroblasts was carried out using the heat resistant siphon reactor. As illustrated in FIG. 3 (A), the entire reactor was sterilely assembled, and the entire device was placed in a $CO_2$ incubator. A 70 ml cell suspension was prepared from human dermal fibroblasts that were cultured in a petri dish and detached therefrom by trypsin treatment. The number of viable cells per ml was $1.4 \times 10^5$ as determined by a cell count. The 70 ml suspension was added to the interior of a Soxhlet tube into which a metal module was provided and left to stand for 30 minutes. Thereafter, the internal liquid was sterilely discharged and the discharged liquid was again added into the siphon reactor. This procedure was repeated three times, the liquid was then sampled and the cell number was measured to give a cell number of $8.0 \times 10^3$. It is considered that 94% of the cells were adsorbed under these stationary conditions by natural contact. Next, the medium (KBM Fibro Assist manufactured by Kohjin Bio) was continuously supplied using the pump so that the siphon function was exhibited, stably and periodically providing medium and air (oxygen) into the metal module. Culturing was continued while the medium was exchanged once every three days. Fibronectin was produced stably and in a large amount as measured by a human fibronectin ELISA kit manufactured by Takara Bio, and it was confirmed that highly efficient substance production was easily and continuously achieved (FIG. 5).

As can be seen in the drawings, the amount of a substance produced per unit volume per day was excellent, and in contrast to a normal petri dish culture, as it is very easy to scale up production, productivity can be improved with a compact device. Furthermore, a method for producing rare or useful substances using human primary cells as the basis for substance production by preparing a continuous production system without the use of complicated equipment having means for supplying oxygen, etc., was demonstrated.

REFERENCE SIGNS LIST

| | |
|---|---|
| 1 | siphon-type cell culture device |
| 11 | liquid storage part |
| 12 | inverted U-shaped tube |
| 13 | medium recovery means |
| 14 | medium supply means |
| 15 | medium discharge means |
| 16 | liquid surface |
| 2 | cell culture part support |
| 21a, 21b, 21c, 21d, 21e, 21f | cell culture part |
| 22 | handle |
| 23 | strut |
| 3 | "shishi odoshi"-type cell culture device |
| 31 | box-shaped body |
| 31a | liquid storage part |
| 31b | bottom part |
| 31c | partition plate |
| 32 | porous polymer film |
| 33 | rotary shaft |
| 34 | bearing |
| 35 | medium recovery means |
| 36 | medium supply means |

Claims

1.   A cell culture device **characterized by** comprising:

a porous polymer film;
a cell culture part that accommodates the porous polymer film;
a liquid storage part that stores the cell culture part therein;
a medium supply means disposed on an upper part of the liquid storage part;
an inverted U-shaped tube communicating with a bottom part of the liquid storage part;
a medium recovery means provided below the other end of the inverted U-shaped tube; and
a medium discharge means disposed on the medium recovery means;

wherein the porous polymer films are a three-layer structure porous polymer film having a surface layer A and a surface layer B, the surface layers having a plurality of pores, and a macrovoid layer sandwiched between the surface layers A and B;

wherein an average pore diameter of the pores present in the surface layer A is smaller than an average pore diameter of the pores present in the surface layer B;

wherein the macrovoid layer has a partition wall bonded to the surface layers A and B, and a plurality of macrovoids surrounded by such a partition wall and the surface layers A and B;

wherein the medium supply means is set at a position higher than the cell culture part, and a top part of the inverted U-shaped tube is set at the same height as the top of the cell culture part or at a position lower than the medium supply means; and

wherein when the liquid surface of the medium supplied to the liquid storage part from the medium supply means reaches the top part of the inverted U-shaped tube, the medium is intermittently discharged into the medium recovery means due to the siphon principle.

2.  A cell culture device **characterized by** comprising:

    a porous polymer film;
    a box-shaped body, one end of which is a liquid storage part, the other end of which is a bottom part, provided with a cell culture part in the liquid storage part that accommodates the porous polymer film;
    a rotary shaft provided in a part at the bottom surface side of the box-shaped body;
    a bearing supporting the rotary shaft;
    a medium supply means disposed above the liquid storage part; and
    a medium recovery means disposed below the liquid storage part;

        wherein the porous polymer films are a three-layer structure porous polymer film having a surface layer A and a surface layer B, the surface layers having a plurality of pores, and a macrovoid layer sandwiched between the surface layers A and B;
        wherein an average pore diameter of the pores present in the surface layer A is smaller than an average pore diameter of the pores present in the surface layer B;
        wherein the macrovoid layer has a partition wall bonded to the surface layers A and B, and a plurality of macrovoids surrounded by such a partition wall and the surface layers A and B; and
        wherein when the medium supplied to the liquid storage part from the medium supply means exceeds the weight of the bottom part of the box-shaped body, the medium from the liquid storage part is intermittently discharged into the medium recovery means, and once the medium is discharged, the box-shaped body returns to the original horizontal position.

3.  The cell culture device according to claims 1 or 2, further comprising:

    a culture liquid discharge line communicating with the medium recovery means at one end; and
    a culture liquid supply line communicating with the medium at one end;
    wherein the other end of the discharge line communicates with the other end of the culture liquid supply line via a pump such that the culture liquid can be circulated.

4.  The cell culture device according to any one of claims 1 to 3, wherein the culture liquid distribution means is a mist supplying nozzle.

5.  The cell culture device according to any one of claims 1 to 4, wherein the porous polymer films are installed in the plane flow path, with the films:

    i) being folded up;
    ii) being wound into a roll-like shape;
    iii) concatenating sheets or pieces thereof with a thread-like structure;
    iv) being tied together into a rope-like shape; and/or
    v) two or more thereof being stacked.

6.  The cell culture device according to any one of claims 1 to 5, wherein the porous polymer films are modularized porous polymer films fitted with a casing;

wherein the modularized porous polymer films fitted with a casing are contained within the casing with:

(i) the two or more independent porous polymer films being aggregated;
(ii) the porous polymer films being folded up;
(iii) the porous polymer films being wound into a roll-like shape; and/or
(iv) the porous polymer films being tied together into a rope-like shape;

wherein the modularized porous polymer films fitted with the casing are installed in the planar flow path.

7. The cell culture device according to any one of claims 1 to 6, wherein the porous polymer film has a plurality of pores having an average pore diameter of 0.01 to 100 $\mu$m.

8. The cell culture device according to any one of claims 1 to 7, wherein an average pore diameter of the surface layer A is 0.01 to 50 $\mu$m.

9. The cell culture device according to any one of claims 1 to 8, wherein the average pore diameter of the surface layer B is 20 to 100 $\mu$m.

10. The cell culture device according to any one of claims 1 to 9, wherein a total film thickness of the porous polymer film is 5 to 500 $\mu$m.

11. The cell culture device according to any one of claims 1 to 10, wherein the porous polymer film is a porous polyimide film.

12. The cell culture device according to claim 11, wherein the porous polyimide film is a porous polyimide film comprising a polyimide derived from tetracarboxylic dianhydride and diamine.

13. The cell culture device according to claim 11 or 12, wherein the porous polyimide film is a colored porous polyimide film that is obtained by molding a polyamic acid solution composition comprising a polyamic acid solution derived from tetracarboxylic dianhydride and diamine, and a coloring precursor, and subsequently heat-treating the resultant composition at 250°C or higher.

14. The cell culture device according to any one of claims 1 to 10, wherein the porous polymer film is a porous polyethersulfone film.

15. A method for culturing a cell which uses the cell culture device according to any one of claims 1 to 14.

**Patentansprüche**

1. Zellkulturvorrichtung, **dadurch gekennzeichnet, dass** sie Folgendes umfasst:

einen porösen Polymerfilm;
ein Zellkulturteil, das den porösen Polymerfilm aufnimmt;
ein Flüssigkeitsspeicherteil, das das Zellkulturteil darin speichert;
eine Mediumzufuhreinrichtung, die an einem oberen Teil des Flüssigkeitsspeicherteils angeordnet ist;
ein umgekehrt U-förmiges Rohr, das mit einem unteren Teil des Flüssigkeitsspeicherteils in Verbindung ist;
eine Mediumrückgewinnungseinrichtung, die unterhalb des anderen Endes des umgekehrt U-förmigen Rohrs bereitgestellt ist; und
eine Mediumablasseinrichtung, die an der Mediumrückgewinnungseinrichtung angeordnet ist;

wobei die porösen Polymerfilme ein poröser Polymerfilm mit dreischichtiger Struktur ist, der eine Oberflächenschicht A und eine Oberflächenschicht B, wobei die Oberflächenschichten eine Vielzahl von Poren aufweisen, und eine Makrokavitätsschicht, die zwischen den Oberflächenschichten A und B liegt, aufweist;
wobei ein durchschnittlicher Porendurchmesser der in der Oberflächenschicht A vorhandenen Poren kleiner ist als ein durchschnittlicher Porendurchmesser der in der Oberflächenschicht B vorhandenen Poren;
wobei die Makrokavitätsschicht eine Trennwand aufweist, die an die Oberflächenschichten A und B gebunden ist, und eine Vielzahl von Makrokavitäten, die von einer solchen Trennwand und den Oberflächenschichten A und B umgeben sind;

wobei die Mediumzufuhreinrichtung an einer höheren Position als das Zellkulturteil angeordnet ist und ein oberes Teil des umgekehrt U-förmigen Rohrs auf der gleichen Höhe wie das obere Teil des Zellkulturteils oder an einer niedrigeren Position als die Mediumzufuhreinrichtung angeordnet ist; und

wobei, wenn die Flüssigkeitsoberfläche des Mediums, das dem Flüssigkeitsspeicherteil von der Mediumzufuhreinrichtung zugeführt wird, den oberen Teil des umgekehrt U-förmigen Rohrs erreicht, das Medium aufgrund des Siphonprinzips intermittierend in die Mediumrückgewinnungseinrichtung entladen wird.

2. Zellkulturvorrichtung, **dadurch gekennzeichnet, dass** sie Folgendes umfasst:

einen porösen Polymerfilm;

einen kastenförmigen Körper, wovon ein Ende ein Flüssigkeitsspeicherteil ist, wovon das andere Ende ein Bodenteil ist, das mit einem Zellkulturteil in dem Flüssigkeitsspeicherteil, der den porösen Polymerfilm aufnimmt, versehen ist;

eine drehbare Welle, die in einem Teil an der Bodenfläche des kastenförmigen Körpers bereitgestellt ist;

ein Lager, das die rotierende Welle trägt;

eine Mediumzufuhreinrichtung, die über dem Flüssigkeitsspeicherteil angeordnet ist; und

eine Mediumrückgewinnungseinrichtung, die unter dem Flüssigkeitsspeicherteil angeordnet ist;

wobei die porösen Polymerfilme ein poröser Polymerfilm mit dreischichtiger Struktur ist, der eine Oberflächenschicht A und eine Oberflächenschicht B, wobei die Oberflächenschichten eine Vielzahl von Poren aufweisen, und eine Makrokavitätsschicht, die zwischen den Oberflächenschichten A und B liegt, aufweist;

wobei ein durchschnittlicher Porendurchmesser der in der Oberflächenschicht A vorhandenen Poren kleiner ist als ein durchschnittlicher Porendurchmesser der in der Oberflächenschicht B vorhandenen Poren;

wobei die Makrokavitätsschicht eine Trennwand aufweist, die an die Oberflächenschichten A und B gebunden ist, und eine Vielzahl von Makrokavitäten, die von einer solchen Trennwand und den Oberflächenschichten A und B umgeben sind; und

wobei, wenn das Medium, das dem Flüssigkeitsspeicherteil von der Mediumzufuhreinrichtung zugeführt wird, das Gewicht des Bodenteils des kastenförmigen Körpers übersteigt, das Medium aus dem Flüssigkeitsspeicherteil intermittierend in die Mediumrückgewinnungseinrichtung entladen wird, und sobald das Medium entladen ist, der kastenförmige Körper in die ursprüngliche horizontale Position zurückkehrt.

3. Zellkulturvorrichtung nach Anspruch 1 oder 2, ferner umfassend:

eine Kulturflüssigkeit-Ablassleitung, die an einem Ende mit der Mediumrückgewinnungseinrichtung verbunden ist; und

eine Kulturflüssigkeit-Zufuhrleitung, die an einem Ende mit dem Medium verbunden ist;

wobei das andere Ende der Ablassleitung über eine Pumpe mit dem anderen Ende der Kulturflüssigkeit-Zufuhrleitung in Verbindung ist, sodass die Kulturflüssigkeit zirkuliert werden kann.

4. Zellkulturvorrichtung nach einem der Ansprüche 1 bis 3, wobei die Kulturflüssigkeit-Verteilungseinrichtung eine Nebelzufuhrdüse ist.

5. Zellkulturvorrichtung nach einem der Ansprüche 1 bis 4, wobei die porösen Polymerfilme in der ebenen Strömungsweg installiert sind, wobei die Filme:

i) zusammengeklappt werden;

ii) in eine rollenartige Form gewickelt werden;

iii) Platten oder Teile davon mit einer fadenartigen Struktur verketten;

iv) zu einer seilartigen Form zusammengebunden werden; und/oder

v) zwei oder mehrere davon gestapelt werden.

6. Zellkulturvorrichtung nach einem der Ansprüche 1 bis 5, wobei die porösen Polymerfilme modularisierte poröse Polymerfilme sind, die mit einer Hülle montiert sind;

wobei die modularisierten porösen Polymerfilme, die mit einer Hülle montiert sind, in der Hülle enthalten sind, wobei:

(i) die zwei oder mehreren unabhängigen porösen Polymerfilme aggregiert werden;

(ii) die porösen Polymerfilme zusammengefaltet werden;

(iii) die porösen Polymerfilme in eine rollenartige Form gewickelt werden; und/oder

(iv) die porösen Polymerfilme zu einer seilartigen Form zusammengebunden sind;

wobei die modularisierten porösen Polymerfilme, die mit der Hülle montiert sind, in dem ebenen Strömungsweg installiert sind.

7. Zellkulturvorrichtung nach einem der Ansprüche 1 bis 6, wobei der poröse Polymerfilm eine Vielzahl von Poren aufweist, die einen durchschnittlichen Porendurchmesser von 0,01 bis 100 $\mu$m aufweisen.

8. Zellkulturvorrichtung nach einem der Ansprüche 1 bis 7, wobei ein durchschnittlicher Porendurchmesser der Oberflächenschicht A 0,01 bis 50 $\mu$m ist.

9. Zellkulturvorrichtung nach einem der Ansprüche 1 bis 8, wobei der durchschnittliche Porendurchmesser der Oberflächenschicht B 20 bis 100 $\mu$m ist.

10. Zellkulturvorrichtung nach einem der Ansprüche 1 bis 9, wobei die Gesamtschichtstärke der porösen Polymerschicht 5 bis 500 $\mu$m ist.

11. Zellkulturvorrichtung nach einem der Ansprüche 1 bis 10, wobei der poröse Polymerfilm ein poröser Polyimidfilm ist.

12. Zellkulturvorrichtung nach Anspruch 11, wobei der poröse Polyimidfilm ein poröser Polyimidfilm ist, umfassend ein von Tetracarbonsäuredianhydrid und Diamin abgeleitetes Polyimid.

13. Zellkulturvorrichtung nach Anspruch 11 oder 12, wobei der poröse Polyimidfilm ein gefärbter poröser Polyimidfilm ist, der durch Formen einer Polyamidsäurelösung-Zusammensetzung erlangt wird, umfassend eine von Tetracarbonsäuredianhydrid und Diamin abgeleitete Polyamidsäurelösung und einen Färbevorläufer, und anschließende Wärmebehandlung der resultierenden Zusammensetzung bei 250 °C oder höher.

14. Zellkulturvorrichtung nach einem der Ansprüche 1 bis 10, wobei der poröse Polymerfilm ein poröser Polyethersulfonfilm ist.

15. Verfahren zum Kultivieren einer Zelle, das die Zellkulturvorrichtung nach einem der Ansprüche 1 bis 14 verwendet.

**Revendications**

1. Dispositif de culture cellulaire **caractérisé en ce qu'**il comprend :

un film polymère poreux ;

une partie de culture cellulaire qui reçoit le film polymère poreux ;

une partie de stockage de liquide qui stocke la partie de culture cellulaire dans celle-ci ;

un moyen d'alimentation en milieu disposé sur une partie supérieure de la partie de stockage de liquide ;

un tube en forme de U inversé communiquant avec une partie inférieure de la partie de stockage de liquide ;

un moyen de récupération de milieu disposé en dessous de l'autre extrémité du tube en forme de U inversé ; et

un moyen de décharge de milieu disposé sur le moyen de récupération de milieu ;

lesdits films polymères poreux étant un film polymère poreux à structure à trois couches possédant une couche de surface A et une couche de surface B, les couches de surface possédant une pluralité de pores, et une couche de macrovides intercalée entre les couches de surface A et B ;

un diamètre de pore moyen des pores présents dans la couche de surface A étant inférieur à un diamètre de pore moyen des pores présents dans la couche de surface B ;

ladite couche de macrovides possédant une paroi de séparation liée aux couches de surface A et B, et une pluralité de macrovides entourés par une telle paroi de séparation et les couches de surface A et B ;

ledit moyen d'alimentation en milieu étant placé au niveau d'une position plus élevée que la partie de culture cellulaire, et une partie supérieure du tube en forme de U inversé étant placée à la même hauteur que le sommet de la partie de culture cellulaire ou au niveau d'une position plus basse que le moyen d'alimentation en milieu ; et

lorsque la surface liquide du milieu fournie à la partie de stockage de liquide à partir du moyen d'alimentation en milieu atteint la partie supérieure du tube en forme de U inversé, ledit milieu étant déchargé par intermittence dans le moyen de récupération de milieu en raison du principe de siphon.

2. Dispositif de culture cellulaire **caractérisé en ce qu'**il comprend :

un film polymère poreux ;
un corps en forme de boîte, dont une extrémité est une partie de stockage de liquide, dont l'autre extrémité est une partie inférieure, dotée d'une partie de culture cellulaire dans la partie de stockage de liquide qui reçoit le film polymère poreux ;
un arbre rotatif prévu dans une partie du côté de la surface inférieure du corps en forme de boîte ;
un palier supportant l'arbre rotatif ;
un moyen d'alimentation en milieu disposé au-dessus de la partie de stockage de liquide ; et
un moyen de récupération de milieu disposé en dessous de la partie de stockage de liquide ;

lesdits films polymères poreux étant un film polymère poreux à structure à trois couches possédant une couche de surface A et une couche de surface B, les couches de surface possédant une pluralité de pores, et une couche de macrovides intercalée entre les couches de surface A et B ;
un diamètre de pore moyen des pores présents dans la couche de surface A étant inférieur à un diamètre de pore moyen des pores présents dans la couche de surface B ;
ladite couche de macrovides possédant une paroi de séparation liée aux couches de surface A et B, et une pluralité de macrovides entourés par une telle paroi de séparation et les couches de surface A et B ; et lorsque le milieu fourni à la partie de stockage de liquide en provenance du moyen d'alimentation en milieu dépasse le poids de la partie inférieure du corps en forme de boîte, ledit milieu de la partie de stockage de liquide étant déchargé par intermittence dans le moyen de récupération de milieu, et une fois que le milieu est déchargé, ledit corps en forme de boîte revenant à la position horizontale d'origine.

3. Dispositif de culture cellulaire selon les revendications 1 ou 2, comprenant en outre :

une conduite de décharge de liquide de culture communiquant avec le moyen de récupération de milieu au niveau d'une extrémité ; et
une conduite d'alimentation en liquide de culture communiquant avec le milieu au niveau d'une extrémité ; ladite autre extrémité de la conduite de décharge communiquant avec l'autre extrémité de la conduite d'alimentation en liquide de culture par l'intermédiaire d'une pompe de sorte que le liquide de culture puisse circuler.

4. Dispositif de culture cellulaire selon l'une quelconque des revendications 1 à 3, ledit moyen de distribution de liquide de culture étant une buse d'alimentation en brouillard.

5. Dispositif de culture cellulaire selon l'une quelconque des revendications 1 à 4, lesdits films polymères poreux étant installés dans le trajet d'écoulement plan, avec les films :

i) qui sont repliés ;
ii) qui sont enroulés en une forme semblable à un rouleau ;
iii) qui enchaînent des feuilles ou des morceaux de celles-ci avec une structure filiforme ;
iv) qui sont attachés ensemble en une forme semblable à une corde ; et/ou
v) qui sont empilés par deux ou plus.

6. Dispositif de culture cellulaire selon l'une quelconque des revendications 1 à 5, lesdits films polymères poreux étant des films polymères poreux modulaires dotés d'une enveloppe ;

lesdits films polymères poreux modulaires dotés d'une enveloppe étant contenus à l'intérieur de l'enveloppe avec :

(i) les deux films polymères poreux indépendants, ou plus, qui sont agrégés ;
(ii) les films polymères poreux qui sont repliés ;
(iii) les films polymères poreux qui sont enroulés en une forme semblable à un rouleau ; et/ou
(iv) les films polymères poreux qui sont liés ensemble en une forme semblable à une corde ;

lesdits films polymères poreux modulaires dotés d'une enveloppe étant installés dans le trajet d'écoulement plan.

**7.** Dispositif de culture cellulaire selon l'une quelconque des revendications 1 à 6, ledit film polymère poreux possédant une pluralité de pores présentant un diamètre de pore moyen de 0,01 à 100 $\mu$m.

**8.** Dispositif de culture cellulaire selon l'une quelconque des revendications 1 à 7, un diamètre de pore moyen de la couche superficielle A étant de 0,01 à 50 $\mu$m.

**9.** Dispositif de culture cellulaire selon l'une quelconque des revendications 1 à 8, ledit diamètre de pore moyen de la couche de surface B étant de 20 à 100 $\mu$m.

**10.** Dispositif de culture cellulaire selon l'une quelconque des revendications 1 à 9, une épaisseur totale de film du film polymère poreux étant de 5 à 500 $\mu$m.

**11.** Dispositif de culture cellulaire selon l'une quelconque des revendications 1 à 10, ledit film polymère poreux étant un film polyimide poreux.

**12.** Dispositif de culture cellulaire selon la revendication 11, ledit film de polyimide poreux étant un film de polyimide poreux comprenant un polyimide dérivé d'un dianhydride tétracarboxylique et d'une diamine.

**13.** Dispositif de culture cellulaire selon la revendication 11 ou 12, ledit film polyimide poreux étant un film polyimide poreux coloré qui est obtenu par moulage d'une composition de solution d'acide polyamique comprenant une solution d'acide polyamique dérivée d'un dianhydride tétracarboxylique et d'une diamine, et un précurseur colorant, et traitement thermique ultérieur de la composition résultante à 250°C ou plus.

**14.** Dispositif de culture cellulaire selon l'une quelconque des revendications 1 à 10, ledit film polymère poreux étant un film poreux de polyéthersulfone.

**15.** Procédé permettant la culture d'une cellule qui utilise le dispositif de culture cellulaire selon l'une quelconque des revendications 1 à 14.

# FIG. 1

FIG. 2

SUPPLY

RETURN

EP 3 489 342 B1

# FIG. 3

(A)

SYPHON-TYPE REACTOR

LID

MEDIUM SUPPLY MEANS

MEDIUM
UPPER LIMIT

U-SHAPED TUBE

MEDIUM
SUPPLY LINE

MAIN BODY PART

CELL CULTURE
MODULE

PUMP

P

MEDIUM
DISCHARGE PORT

MEDIUM
DISCHARGE TUBE

MEDIUM

MEDIUM SUPPLY LINE

MEDIUM DISCHARGE LINE

MEDIUM RESERVOIR

(B)

MODULARIZED POROUS
POLYMER FILM

POROUS POLYMER FILM

CASING

26

FIG. 4

EP 3 489 342 B1

# FIG. 5

(ng/ml/day)

# FIG. 6

MESH SURFACE

LARGE PORE SURFACE

APPARENT MEMBER VOLUME

MEMBER VOLUME CONTAINING CELL SURVIVAL ZONE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2003054174 A
- WO 2010038873 A
- JP 2011219585 A
- JP 2011219586 A
- WO 2015012415 A
- WO 2009123349 A
- JP 2009057041 W